# EUROPEAN PATENT APPLICATION

(11) **EP 4 414 453 A1**
(43) Date of publication of application: **14.08.2024**
(21) Application number: 23155584.8
(22) Date of filing: 08.02.2023
(51) Int. Cl.: C12N 15/115, B82Y 15/00, G01N 33/53, C07D 487/22

(54) **AFFINITY REAGENT FOR REVERSIBLY BINDING TO A MOLECULAR TARGET**

(71) Applicant: Leica Microsystems CMS GmbH, 35578 Wetzlar (DE)
(72) Inventor: Alsheimer, Soeren, 35578 Wetzlar (DE)
(74) Representative: Schaumburg und Partner Patentanwälte mbB

(57) **Abstract**

An affinity reagent (104) for reversibly binding to a molecular target (108) comprises a guest-modified aptamer (106) which includes at least one guest molecule (102) adapted for complex formation with at least one host molecule (100). The guest-modified aptamer (106) is structurally alterable by said complex formation to either associate with or dissociate from the molecular target (108).

## Description

### Technical field

The invention relates to an affinity reagent, a structure and a device comprising a plurality of affinity reagents, a reagent kit, and a method for reversibly binding affinity reagents to molecular targets.

### Background

In the field of life sciences, it is vital to precisely detect the presence of analytes, i.e. molecular targets in biological samples such as tissue samples and cell cultures, environmental or soil samples, water samples, and the like. Molecular targets must also be detected in diagnostic procedures such as in solid or liquid biopsy, in a sample prepared from either, or a lysate or extract from such a sample. This can be performed by introducing markers into the sample that bind to the molecular targets. Such a marker typically comprises an affinity reagent that attaches to the structure in question and a detectable label such as a fluorescent dye, a metal tag, a polyyne label, a nucleic acid label (barcode), or an enzyme that is either directly conjugated to the affinity reagent or attached to the affinity reagent by means of a secondary affinity reagent. There are various techniques for analyzing samples prepared in this way.

Affinity reagents comprise chemically heterogeneous groups including for example antibodies. Currently, there is a large number of different antibodies which are widely used for various detection applications in life science research, diagnostics, and therapeutic applications. However, a typical antibody is relatively large which affects its ability to penetrate a sample.

Aptamers, which can also be used as affinity reagents, are roughly estimated to be about ten times smaller than conventional antibodies. In many applications such as tissue staining, a small size of the affinity reagent is beneficial in terms of sample penetration. Furthermore, compared to antibodies, aptamers are relatively easy and inexpensive to produce. Thus, aptamers are typically selected from libraries of oligonucleotides in a process called SELEX (systematic evolution of ligands by exponential enrichment) which is a fully in vitro process as opposed to antibody discovery and production.

Against this background, it would be conceivable to make greater use of aptamers as affinity reagents in life sciences. In particular, applications are conceivable in which an affinity reagent repeatedly associates to and dissociates from a molecular target, such as in cycling staining of samples imaged by fluorescence microscopy. In principle, it would be possible to reverse binding of aptamers by melting after imaging. However, the high temperatures required are incompatible with cyclical staining, and interfering fluorescence background would increase with the number of cycles.

An approach to induce direct dissociation of DNA duplex structures is described in the article of L. Xiao et al., "Controllable DNA hybridization by host-guest complexation-mediated ligand invasion", Nature Communications 13, 5936 (2022).

### Summary

It is an object to provide a widely applicable affinity reagent which is able to readily penetrate a sample and to reversibly bind a molecular target.

The afore-mentioned object is achieved by an affinity reagent according to claim 1. Advantageous embodiments are defined in the dependent claims and the following description.

An affinity reagent for reversibly binding to a molecular target comprises a guest-modified aptamer which includes at least one guest molecule adapted for complex formation with at least one host molecule. The guest-modified aptamer is structurally alterable by said complex formation to either associate with or dissociate from the molecular target.

The proposed affinity reagent is based on an aptamer which is typically formed of a short nucleic acid sequence. The aptamers can reversibly fold into and out of a three-dimensional (3D) structure. By altering its three-dimensional structure, the aptamer is able to associate with and dissociate from a molecular target.

The aptamer includes at least one guest molecule which is configured to be accommodated by a host molecule. The proposed solution takes advantage of the finding that host-guest complexation-mediated ligand invasion can be used as a principle to disrupt nucleic acid duplexes as described in the afore-mentioned article of Xiao et al., "Controllable DNA hybridization by host-guest complexation-mediated ligand invasion", Nature Communications 13, 5936 (2022), which is incorporated herein by reference. The inventor has recognized that this principle, where the guest-host complexation leads to strand invasion, can be combined with the concept of aptamers to create controllable aptamer-based affinity reagents which can be used to bind and release molecular targets in response to changes in host molecule concentration.

More specifically, it is proposed to use aptamers that comprise at least one nucleotide or nucleoside that is modified by the guest molecule, wherein the positioning of the guest molecule is selected such that the complexation of the guest molecule with the host molecule alters the structure of the aptamer in a manner that either abolishes or enables binding of the molecular target.

The affinity reagent is widely applicable in the field of life sciences with a number of advantages. Due to its smaller size, which is only about one tenth of the size of a typical antibody, the guest-modified aptamer penetrates tissue samples better. Aptamers are typically short, single-stranded DNA or RNA oligonucleotides (about 20 to 80 nucleotides in length corresponding to about 6 to 30 kDa). This puts the aptamers in a similar size range to nanobodies which are antibody fragments derived from llama alpaca or shark antibodies. Such a small size is desirable, for example, when staining tissue for microscopy, as the penetration of antibodies is severely limited due to their size. In particular, the affinity reagent enables cyclical labelling of molecular targets with aptamers. For example, cyclical labelling is applied in multiplexed ("high-plex") fluorescence microscope, where a large number of analytes are identified. The aptamer-based affinity reagent enhances label penetration into the tissue and allows high-plex analysis in spatial biology applications. Time-to-result is reduced, and staining quality is improved. In therapeutic applications, the small size allows rapid renal clearance. Using bulky moieties like polyethylenglycol, renal clearance rates can be tuned to match applicative requirements.

Furthermore, the guest-modified aptamer can be developed and produced based on the afore-mentioned SELEX process which is less complex and inexpensive compared to antibody production. The lower cost of producing test reagents and the ease of functionalization make it possible to offer a range of different assay formats.

Moreover, aptamers can be easily functionalized. Functionalization may be desired in certain assays such ELISA-style assays or when aptamers shall be used for affinity purification.

The controllable, aptamer-based affinity reagent can be of great use in a wide range of applications. For example, the affinity reagent can be used to create responsive biomaterials, biosensors that can be recycled, reagents for cyclical staining processes, dynamic association with and dissociation from molecular targets in super-resolution microscopy, and affinity chromatography resins that can be easily regenerated under mild conditions.

Conjugates of aptamers to peptides such as cytokines or drugs are being investigated in clinical trials. Aptamers are therefore an interesting alternative to antibodies in life science research, diagnostics, and therapeutic applications. Aptamers can be used as an alternative in applications where antibodies are used. Due to their particular properties, size, and nucleic acid sequence backbone, the range of aptamer applications may be even broader than that of antibodies.

As used herein, a molecular target refers to an analyte that is, for instance, a protein, an RNA sequence, e.g. the mRNA of a specific gene, a peptide, e.g. somatostatin, a DNA sequence, e.g. a genetic locus or element, a metabolite, e.g. lactic acid, a hormone, e.g. estradiol, a neurotransmitter, e.g. dopamine, a vitamin, e.g. cobalamine, a micronutrient, e.g. biotin, a metal ion, e.g. metal and heavy metal ions like Cd(II), Co(II), Pb(II), Hg(II), U(VI).

The guest-modified aptamer may include natural nucleic acids such as DNA and RNA or synthetic nucleic acids such as XNA. The guest-modified aptamer may also be based on a peptide backbone. Furthermore, the guest-modified aptamer may also refer to a derivative of an aptamer such as an aptabody which includes modifications typically found on proteins such as glycosylation.

The affinity reagent can be used in a variety of samples such as biological samples including blood, serum, plasma, tissue, bodily fluids (e.g. lymph, saliva, semen, interstitial fluid, cerebrospinal fluid), feces, solid biopsy, liquid biopsy, explants, whole embryos (e.g. zebrafish, Drosophila), entire model organisms (e.g. zebrafish larvae, Drosophila embryos, C. elegans), cells (e.g. prokaryotes, eukaryotes, archea), multicellular organisms (e.g. Volvox), suspension cell cultures, monolayer cell cultures, 3D cell cultures (e.g. spheroids, tumoroids, organoids derived from various organs such as intestine, brain, heart, liver, etc.), a lysate of any of the aforementioned, a virus. A sample shall further refer to a volume surrounding a biological sample such as in assays, where secreted proteins like growth factors, extracellular matrix constituents are being studied. The extracellular environment surrounding a cell up to a certain assay-dependent distance is also referred to as a sample. Specifically, affinity reagents brought into such a surrounding volume are referred to in the sense of this document as being introduced into the sample.

Preferably, the guest-modified aptamer has a single-stranded structure which is alterable by the complex formation between a folded state and an unfolded state. In the folded state, the single strand is hybridized, i.e. bound to itself. In contrast, in the unfolded state, the single strand is unbound.

The guest-modified aptamer may be configured to associate with the molecular target in the folded state and to dissociate from the molecular target in the unfolded state. In this embodiment, the folded state represents a 3D structure in which the single-stranded aptamer is enabled to bind the molecular target with high affinity and specificity.

For example, the guest molecule of the guest-modified aptamer may be adapted for complex formation with a cucurbituril, which is a suitable choice of host molecule. Cucurbituril is a macrocyclic molecule made of glycoluril monomers (C₄H₂N₄O₂) linked by methylene bridges (- CH₂-). The oxygen atoms of the molecule are located along the edges of the band and are tilted inwards, forming a partly enclosed cavitity. Cucurbituril may be written as cucurbit[n]uril, where n is the number of glycoluril units. Preferably, cucurbit[7]uril may be used as host molecule. Cucurbiturils have carbonyl-rich portals and hydrophobic cavities. The residence time of the guest molecule inside the cavity depends strongly on the guest molecule in question. Recent work by Lambert et al. 2022 suggest that a number of ligands binding to the outside of the portal can modify the entry and exit of guest molecules offering further possibilities to fine-tune systems that involve huest-gost complexation as a control element. The afore-mentioned reference is Lambert, H., Castillo Bonillo, A., Zhu, Q. et al., "Supramolecular gating of guest release from cucurbit[7]uril using de novo design", npj Comput Mater 8, 21 (2022). https://doi.org/10.1038/s41524-022-00702-0.

In case that a cucurbituril, e.g. cucurbit[7]uril, is used a host molecule, a suitable guest molecule may be selected from a group consisting of 1-adamantanemethylamine, ferrocenyl methylamine, 1,4-benzenedimethanamine, and 4-tert-butylbenzylamine as demonstrated in the afore-mentioned article of Xiao et al.

Further guest-molecules, which are known to complexate with cucurbit[n]uril include several commonly used fluorescent dyes such as cyanine dyes. It is known that these dyes may complexate with cucurbit[n]uril and do so in 1:1, 1:2, 1:4 ratios depending on the size of the chromophore and size of the cavity. In addition to fluorescent dyes a number of molecules have been found to complexate with different cucurbiturils.

Preferably, the affinity reagent comprises at least one detectable label which may be directly or indirectly bound to the guest-modified aptamer.

Such a label can be detected using an image-based readout or a non-image-based readout. An image-based readout may be acquired by means of a microscope such as a point-scanning confocal microscope, a widefield microscope, in particular a light sheet fluorescence microscope, a light field microscope and a stereo microscope. A non-image-based readout may be acquired by means of a cytometer or a flow-through based readout device having a point detector or a line detector. Furthermore, a readout may be discrete in nature such as a single acquisition of an emission spectrum or an image stack. Moreover, a readout data stream may be provided, such as in a point-scanning confocal microscope or a cytometer which operates continuously. A readout may also be a sequence of images, such as a spectral or hyperspectral image stack, wherein fluorescence emission of different wavelength bands is recorded in each image.

In a particularly preferred embodiment, the label may comprise at least one polyyne. Polyynes are a class of molecules that provide a detectable Raman signal and can be used in imaging or non-imaging based Raman readouts, as e.g. described in Hu F., Zeng C, Long R, Miao Y, Wei L, Xu Q, Min W., "Supermultiplexed optical imaging and barcoding with engineered polyynes", Nat Methods. 2018 Mar;15(3):194-200. doi: 10.1038/nmeth.4578. Epub 2018 Jan 15. PMID: 29334378; PMCID: PMC5831481.

Furthermore, the label may comprise a metal tag.

Furthermore, the label may comprise an enzyme such as horseradish peroxidase (HRP), which may be used to catalyze chromogenic reaction, reporter deposition, or generate a detectable signal through other means (e.g. bioluminescence).

Furthermore, the label may comprise a dye, in particular a fluorescent dye, a fluorophore and a fluorochrome. Specifically, a fluorescent dye denotes a fluorescent chemical compound or structure and may be one of the following: a fluorescent organic dye, a fluorescent quantum dot, a fluorescent dyad, a fluorescent carbon dot, a graphene quantum dot or another carbon-based fluorescent nanostructure, a fluorescent protein, a fluorescent DNA origami-based nanostructure.

Organic fluorescent dyes may also include derivatives of the following: xanthene (e.g. fluorescein, rhodamine, Oregon green, Texas), cyanine (e.g. cyanine, indocarbocyanine, oxacarbocyanine, thiacarbocyanine, merocyanine), squaraine rotaxane derivatives, naphthalene, coumarin, oxadiazole, anthracene (anthraquinones, DRAQ5, DRAQ7, CyTRAK Orange), pyrene (cascade blue), oxazine (Nile red, Nile blue, cresyl violet, oxazine 170), acridine (proflavine, acridine orange, acridine yellow), arylmethine (auramine, crystal violet, malachite green), tetrapyrrole (porphin, phthalocyanine, bilirubin), dipyrromethene (BODIPY, aza-BODIPY), a phosphorescent dye, or a luminescent dye.

The following trademark groups designate commercially available fluorescent dyes which may be used as labels, including dyes that belong to different chemical families such as CF dye (Biotium), DRAQ and CyTRAK probes (BioStatus), BODIPY (Invitrogen), EverFluor (Setareh Biotech), Alexa Fluor (Invitrogen), Bella Fluore (Setareh Biotech), DyLight Fluor (Thermo Scientific), Atto and Tracy (Sigma-Aldrich), FluoProbes (Interchim), Abberior Dyes (Abberior Dyes), Dy and MegaStokes Dyes (Dyomics), Sulfo Cy dyes (Cyandye), HiLyte Fluor (AnaSpec), Seta, SeTau and Square Dyes (SETA BioMedicals), Quasar and Cal Fluor dyes (Biosearch Technologies), SureLight Dyes (Columbia Biosciences), Vio Dyes (Milteny Biotec).

Fluorescent proteins that can be used as labels may be members of the green fluorescent protein (GFP) family including GFP and GFP-like proteins (e.g DsRed, TagRFP) and their (monomerized) derivatives (e.g., EBFP, ECFP, EYFP, Cerulaen, mTurquoise2, YFP, EYFP, mCitrine, Venus, YPet, Superfolder GFP, mCherry, mPlum).

In particular, fluorescent proteins may be used whose absorbance or emission characteristics change upon binding of a ligand such as BFPms1 or in response to changes in the environment such as redox-sensitive roGFP or pH-sensitive variants. Furthermore, labels may include derivatives of cyanobacterial phycobiliprotein small ultra red fluorescent protein smURFP as well as fluorescent protein nanoparticles that can be derived from smURFP. An overview of usable fluorescent proteins can be found in Rodriguez et al. 2017, Trends Biochem Sci. 2017 Feb; 42(2): 111-129.

Further, a label may include a fluorescent quantum dot, in particular a fluorescent carbon dot, a fluorescent graphene quantum dot, a fluorescent carbon-based nanostructure as described in Yan et al., Microchimica Acta, (2019) 186: 583, and Iravani and Varma, Environ Chem Lett., (2020) Mar 10: 1-25.

Further, a label may include a fluorescent polymer dot (Pdot) or nanodiamond, a fluorescent dyad such as a dyad of a perylene antenna, and a triangelium emitter as described in Kacenauskaite et al., J. Am. Chem. Soc. (2021), 143, 1377-1385.

A label may also include an organic dye, a dyad, a quantum dot, a polymer dot, a graphene dot, a carbon-based nanostructure, a DNA origami-based nanostructure, a nanoruler, a polymer bead with incorporated dyes, a fluorescent protein, an inorganic fluorescent dye, a SMILE, or a microcapsule filled with any of the aforementioned substances.

A label may further include to a Forster resonance energy transfer (FRET) pair having at least one fluorescent dye as FRET donor and at least one fluorescent dye as a FRET acceptor, or a FRET-triple, which is used to generate a three component FRET. In particular, the FRET pair or triplet may be connected by a complementary linker or by a linking element.

According to another aspect, as structure is provided which comprises a plurality of affinity reagents as described above. The structure includes at least one of a DNA nanostructure, a gel, a polymer, a chromatography resin, and a magnetic particle.

According to another aspect, a device is provided which comprises a plurality of affinity reagents as described above. The device includes at least one of a biosensor which is a sensor made of a responsive biomaterial including the affinity reagents described above. Preferably, the sensor is configured as a FRET sensor. The device may also include an electrical circuit comprising material which includes the affinity reagents.

According to another aspect, a reagent kit is provided which comprises at least one host molecule and at least one affinity reagent for reversibly binding to a molecular target. The affinity reagent comprises a guest-modified aptamer which includes at least one guest molecule adapted for complex formation with the at least one host molecule. The guest-modified aptamer is structurally alterable by the complex formation to either associate with or dissociate from the molecular target.

In a preferred embodiment, the reagent kit comprises a solid support. Such a solid support may include nanoarrays, DNA bricks, DNA origami, polystyrene beads, magnetic beads, or chromatography resin.

According to a preferred embodiment, the reagent kit comprises a flow cell which may be used, for example, for DNA sequencing.

According to another aspect, a method is provided for reversibly binding affinity reagents to molecular targets, each affinity reagent comprising a guest-modified aptamer which includes at least one guest molecule adapted for complex formation with at least one host molecule, the guest-modified aptamer being structurally alterable by the complex formation to either associate with or dissociate from the respective molecular target. The method comprises the following steps: associating the affinity reagents with the molecular targets at a first concentration of the host molecules; and dissociating the affinity reagents from the molecular targets at a second concentration of the host molecules, wherein the second concentration is different from the first concentration.

This method allows the affinity reagent association and dissociation to be controlled based on the host molecule concentration. Thus, the host-guest complexation-mediated ligand invasion, which is used to disrupt nucleic acid duplexes, is relatively easy to adjust by concentration alone.

Preferably, the method further comprises a step of detecting at least one label included in each affinity reagent after the affinity reagent is associated with the molecular target and before the affinity reagent is dissociated from the molecular target. In this step, the labels specified above can be used. Furthermore, an image-based readout or a non-imaged-based readout may be applied.

Preferably, the method further comprises a step of removing the affinity reagents from a sample containing the molecular targets after the affinity reagents are dissociated from the molecular targets. In this way, the sample can be refreshed with new affinity reagents.

According to a preferred embodiment, a sequence of the steps of associating, detecting, dissociating, and removing is cyclically repeated. By doing so, a readout sequence can be generated in several successive steps.

For example, based on an image-based readout using a fluorescence microscope, a readout sequence may be generated as follows: In a first round, the affinity reagents including distinct optical labels are bound to the molecular targets by adjusting the host molecule concentration to a value that allows the affinity reagents to associate with the molecular targets. Subsequently, the optical labels present in a readout volume of the sample are illuminated with excitation light and fluorescence light emitted from the distinct optical labels is detected. In this process, the detected fluorescence light may be separated by the readout device into multiple color channels, each channel corresponding to one label species. After detecting the fluorescence light, the affinity reagents with the optical labels are separated from the molecular targets by adjusting the host molecule concentration to a value that allows the affinity reagents to dissociate from the molecular targets. Then, the affinity reagents are removed from the sample, completing the first round. Further rounds are executed in the same way to generate the readout sequence.

After obtaining a readout sequence as described above, presence or absence of an optical label in a readout volume can be assessed qualitatively and/or quantitatively, wherein qualitatively refers to calling an optical label present (presence calling) in the readout volume. A qualitative assessment may be performed by determining whether or not the intensity in the corresponding color channel is above a certain user-defined threshold. The threshold may be a fixed threshold, a fixed channel-specific threshold, or a dynamically adjusted threshold. Furthermore, the threshold may be a combination of different thresholds such as an intensity threshold and a statistical confidence in the dye separation result. A quantitative assessment may involve an assignment of a relative intensity value or an absolute number of molecules to the detected signal.

The readout sequences may be acquired as times series which are continuous at each position of an imaging area of the sample. Further, the readout sequences may also be acquired in cycles or iteratively in a process called "code swapping". This process depends on an inactivation of bound labels (wherein such a label may also be a combinatorial label comprising several distinct labels) from a specific round n, washing out the unbound or inactivated labels from round n before a relabeling with at least some of the labels that have been reassigned to at least some of the affinity reagents in round n+1. This may be referred to as interround "code swapping", because codes represented by the labels are reassigned from one round to the next.

Although an interround code swapping as described above may by a suitable procedure depending on the specific application, it is to be noted that the proposed solution removes the necessity to perform a cyclical process so that code swapping can also be performed in a way which is called "intraround". For example, an intraround code swapping can be performed dynamically. In a dynamic intraround code swapping, a plurality of distinct combinatorial labels assigned to a particular affinity reagent are allowed to dynamically associate and dissociate with the oligonucleotide-barcode of said affinity reagent. Alternatively, a repeated intraround code swapping can be performed, wherein a plurality of distinct combinatorial labels assigned to a particular affinity reagent are allowed to repeatedly associate and dissociate with the oligonucleotide-barcode of said affinity reagent.

In principle, dynamic intraround code swapping can be performed by using DNA exchange imaging technology, controllable DNA hybridization strategies as well as simply by raising the temperature close to the melting temperature of the oligonucleotide-barcode-attachment portion of the linker interaction, where the association constant will largely depend on the length of the complementary stretch, number of mismatches (if any are introduced) and the ionic strength of the buffer. It is to be noted that raising the temperature close to the melting point can be performed with oligonucleotide-based linker or backbone structures of combinatorial labels, if they have been stabilized before using a suitable strategy such as for example ligation, or UV-mediated cross-linking, or chemical cross-linking. However, a preferred way to implement dynamic intraround code swapping is to use the affinity reagents as described herein and to control hybridization by keeping the host molecule concentration at a suitable concentration. Thus, in dynamic intraround code swapping different combinatorial labels assigned to a particular affinity reagent associate and dissociate randomly with the respective oligonucleotide-barcode.

In principle, repeated intraround code swapping can be performed using temperature cycling, i.e. melting and reannealing, or by using photoswitchable NCDA as described by Dohno et al., Journal of the American Chemical Society (2007), 129 (39), 11898-11899). However, a preferred way to implement repeated intraround code swapping is to use the affinity reagents as described herein and to control hybridization by changing the host molecule concentration repeatedly or by keeping the concentration of host molecule in the zone of transient association/dissociation, i.e. at a medium concentration.

Both, dynamic intraround code swapping and repeated intraround code swapping may either be performed by introduction all necessary reagents to the sample before the acquisition of readout traces or by applying at least some of the reagents or modifying at least some of the physical parameters such as illumination with UV light, e.g. 360nm, 430nm light, or the temperature during the acquisition of the readout traces.

In this respect, a readout trace refers to a time series acquisition of a readout sequence. The difference between acquiring multiple readout sequences in a cyclical process and acquiring a readout trace is that a readout trace is not interrupted by pauses for inactivation of labels, washing and restaining. In a preferred embodiment, a biological sample may be analysed by performing tile scans and recording short time series at each tile. Such readout traces may be acquired with intervals in the ms-min range or as a continuous acquisition (streaming) of particular position (tile). Assuming an image-based readout by means of a microscope, a readout trace may also be performed with regard to a given volume by acquiring time series at a certain XY position while moving the sample relative to the readout in Z. In a particularly preferred embodiment, a readout is used that comprises an object plane orientation which is at an angle relative to the (optical) surface of a sample carrier, e.g. a microscope slide, an imaging well chamber, or a well plate bottom. This may in particular be a microscope based on a SCAPE or OPM configuration.

### Short Description of the Figures

Hereinafter, specific embodiments are described referring to the drawings, wherein:
- Figure 1: is a schematic diagram showing a host molecule and a guest molecule which is adapted to form a complex with the host molecule depending on the concentration thereof;
- Figure 2: is a schematic diagram illustrating a guest-modified aptamer which folds and unfolds in response to the host molecule concentration to associate with and dissociate from a molecular target, respectively;
- Figure 3: is a schematic diagram showing fractions of associated and dissociated guest-modified aptamers as a function of the host molecule concentration;
- Figure 4: is a schematic diagram illustrating a method for interround code swapping according to an embodiment;
- Figure 5: is a schematic diagram illustrating a method for intraround code swapping according to an embodiment;
- Figure 6: is a schematic diagram illustrating a method for interround code swapping according to another embodiment;
- Figure 7: is a schematic diagram illustrating a method for intraround code swapping according to another embodiment;
- Figure 8: is a schematic diagram illustrating an example in which the affinity reagent is used for affinity purification;
- Figure 9: is a schematic diagram illustrating a FRET sensor including the affinity reagent; and
- Figure 10: is a schematic diagram illustrating an example in which the affinity reagent is used for building a responsive biomaterial.

### Detailed Description

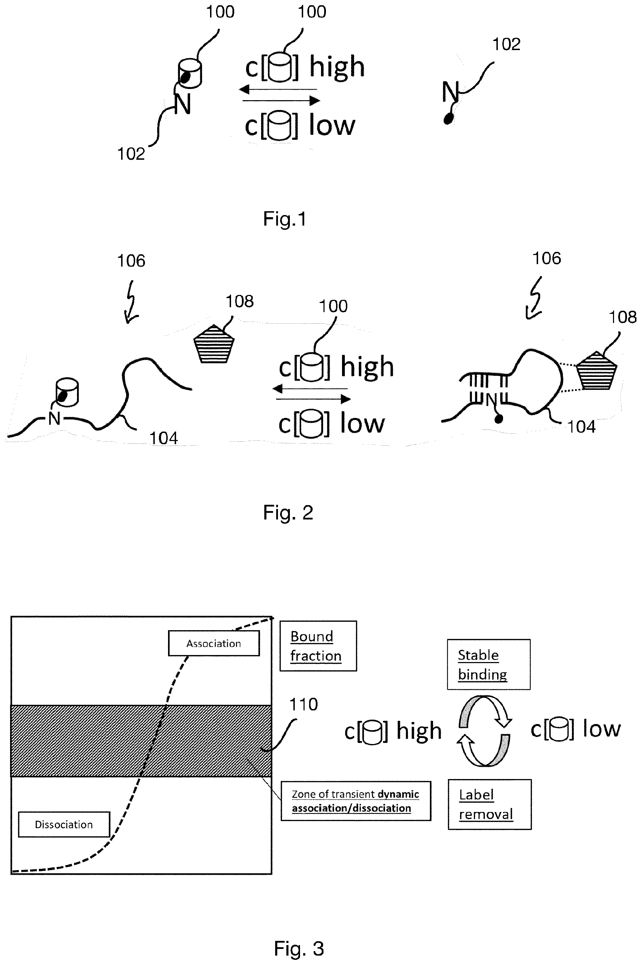

Figure 1 is a schematic diagram showing a host molecule 100 and a guest molecule 102 which is adapted to form a complex with the host molecule 102.

According to the embodiment shown in Figure 1, it is assumed that the host molecule 100 is a curcubituril, e.g. curcubit[7]uril. Accordingly, the guest molecule 102 is selected such it is able to form a complex with curcubituril. For this purpose, the guest molecule 102 may be selected from a group consisting of 1-adamantanemethylamine, ferrocenyl methylamine, 1,4-benzenedimethanamine, and 4-tert-butylbenzylamine.

As indicated in Figure 1 by opposite arrows, the complex formation depends on the concentration c of the host molecule 100. In this example, complex formation increases when the host molecule concentration becomes larger as illustrated by the arrow pointing from the right to the left in Figure 1. Likewise, complex formation decreases when the host molecule concentration becomes smaller as illustrated by the arrow pointing from the left to the right in Figure 1.

Figure 2 illustrates a host-guest complexation-mediated ligand invasion that can be used to reversibly alter a three-dimensional structure of an affinity reagent 104 comprising an aptamer 106 in response to changes in the host molecule concentration c. The aptamer 106 may be a single-stranded DNA, RNA or XNA oligonucleotide which is modified to include the guest molecule 102. More specifically, the aptamer 106 includes at least one nucleotide or nucleoside, that is modified by the guest molecule 102, at a suitable position of the DNA, RNA or XNA sequence. Such a guest-modification may be achieved by incorporating the guest molecule 102 into the duplex structure of the self-hybridized aptamer 106 that is either part of or close to a binding site/pocket forming portion of the aptamer 104. Thus, the guest-modified aptamer 106 is enabled to fold correctly and to bind a molecular target 108 in case that host molecule concentration c is sufficiently low, as illustrated on the right of Figure 2.

On the other hand, if the host molecule concentration c is high, the guest-molecule 102 incorporated into the aptamer 106 forms a complex with the host molecule 100, and proper folding of the aptamer 106 is prohibited. In other words, the guest-modified aptamer 106 unfolds at a high host molecule concentration c with result that the guest-modified aptamer 106 is unable to bind to the target molecule 108, as illustrated on the left of Figure 2.

Accordingly, the affinity reagent 104 comprising the guest-modified aptamer 106 shown in Figure 2 can be used to either associate with or to dissociate from the molecular target 108 depending on the host molecule concentration c. In particular, the host-guest complexation can be used to control nucleic acid duplex formation. In the absence or at a low host molecule concentration, the guest molecule 102 does not impair duplex formation. Thus, a normal melting temperature is observed for the guest-modified aptamer 106. In contrast, at a high host molecule concentration c, guest-host complexation occurs, which in turn leads to ligand-invasion into the duplex thereby either disrupting an existing duplex, abolishing duplex formation, and/or lowering the melting temperature of the duplex significantly.

While in the specific embodiment of Figure 2 it is assumed that association occurs in the folded state and dissociation occurs in the unfolded state, a reverse approach is also possible. This can be achieved by placing the guest-molecule at a site in the aptamer 106 that allows proper folding only to occur upon disruption of a certain duplex. This may also be a feature that may be specifically added to a particular aptamer by using strands that compete with a another stretch of the aptamer's sequence.

Figure 3 is a diagram showing the fractions at which the affinity reagent 104 including the guest-modified aptamer 106 associates with or dissociates from the target molecule 108 as a function of the host molecule concentration c.

According to Figure 3, the association of the affinity reagent increases slowly at low host molecule concentrations c. Subsequently, the association increases more steeply until it reaches a transient zone 110 where association and dissociation are essentially in balance. Toward higher host molecule concentrations c, the association increases more gently again.

The characteristic shown in Figure 3 can be used in a variety of applications for reversibly binding the affinity reagent 104 to the molecular target 108.

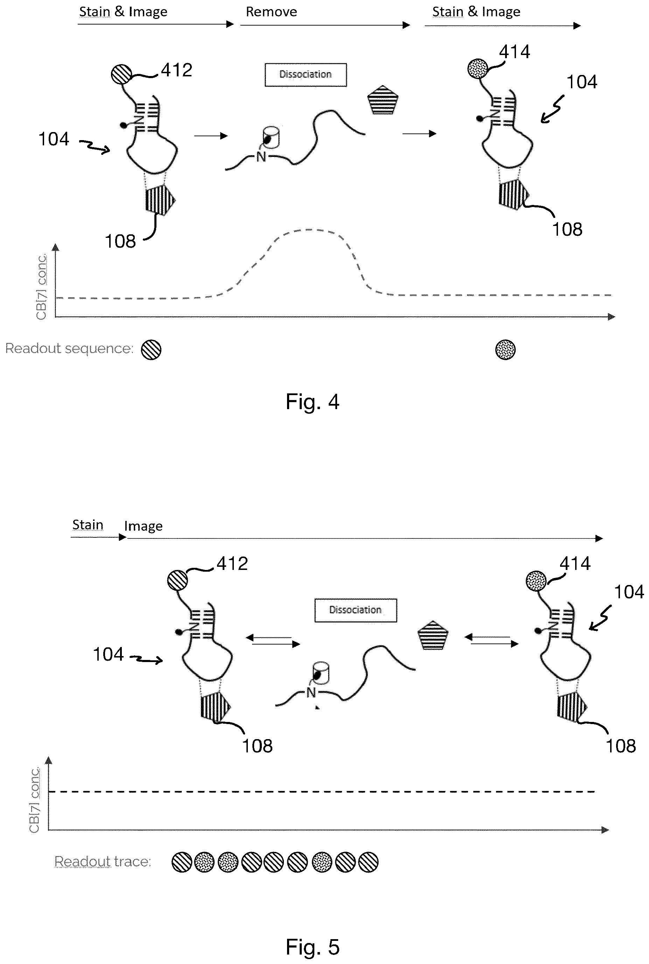

Figure 4 illustrates an exemplary application in which an interround code swapping is performed. In this example, it is assumed that the affinity reagent 104 includes a single optical label, e.g. a fluorescent dye which can be detected based on an imaged-based readout using a fluorescence microscope.

In the example of Figure 4, the sample is stained to provide the affinity reagent 104 with a first optical label 412. In a state in which the host molecule concentration c is low, the affinity reagent 104 including the first label 412 associates with the molecular target 108. In the associated state, an optical readout is executed in which the sample is imaged by the fluorescence microscope. Subsequently, the host molecule concentration c is increased to remove the affinity reagent 104 with the first label 412 from the molecular target 108. This completes a first round. A second round of staining, imaging and removing is then executed, wherein the first label 412 is replaced by a second label 414. By executing multiple rounds, interround swapping is performed to generate a single label-based readout sequence.

Figure 5 illustrates another exemplary application in which a dynamic intraround code swapping is performed. Compared to the example of Figure 4, the process in Figure 5 is modified by keeping the host molecule concentration at a suitable value within the transient zone 110 of the characteristic shown in Figure 3. Furthermore, the sample is stained only once with multiple labels including the first and second labels 412, 414. Subsequently, an optical readout is continuously executed. As a result, a readout trace is generated as shown in Figure 5.

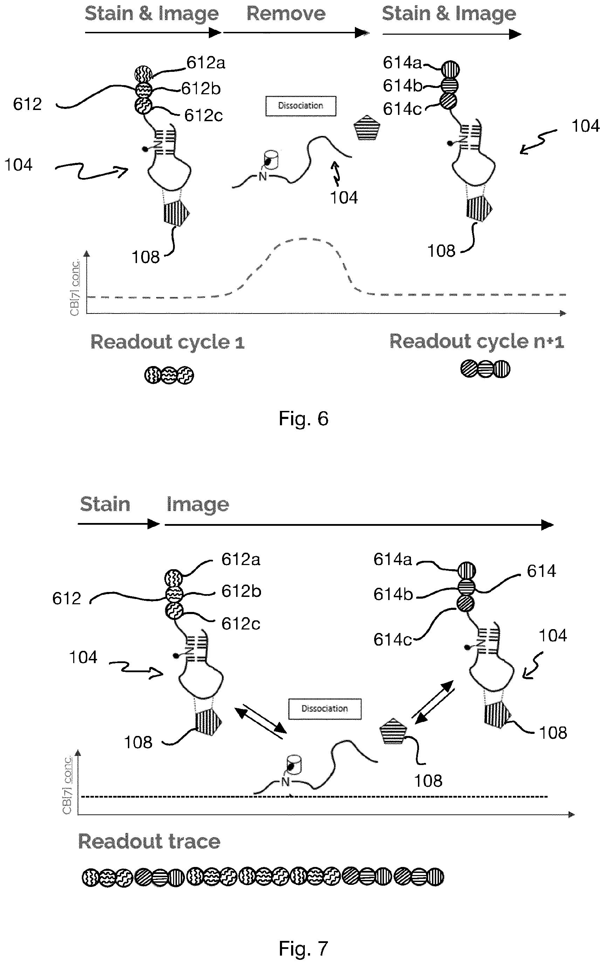

Figure 6 illustrates another exemplary application which is based on the principle of interround code swapping shown in Figure 4. However, compared to the example of Figure 4, the process in Figure 6 is modified by a combinatorial label 612, 614 which includes multiple distinct labels 612a-c, 614a-c replacing the single label 412, 414. By executing multiple rounds, interround swapping is performed to generate a combinatorial label-based readout sequence.

Figure 7 illustrates another exemplary application which is based on the principle of intraround code swapping shown in Figure 5. However, compared to the example of Figure 5, the process in Figure 7 is modified by the combinatorial label 612, 614 which includes the multiple distinct labels 612a-c, 614a-c. By executing an optical readout continuously, a readout trace is generated as shown in Figure 7.

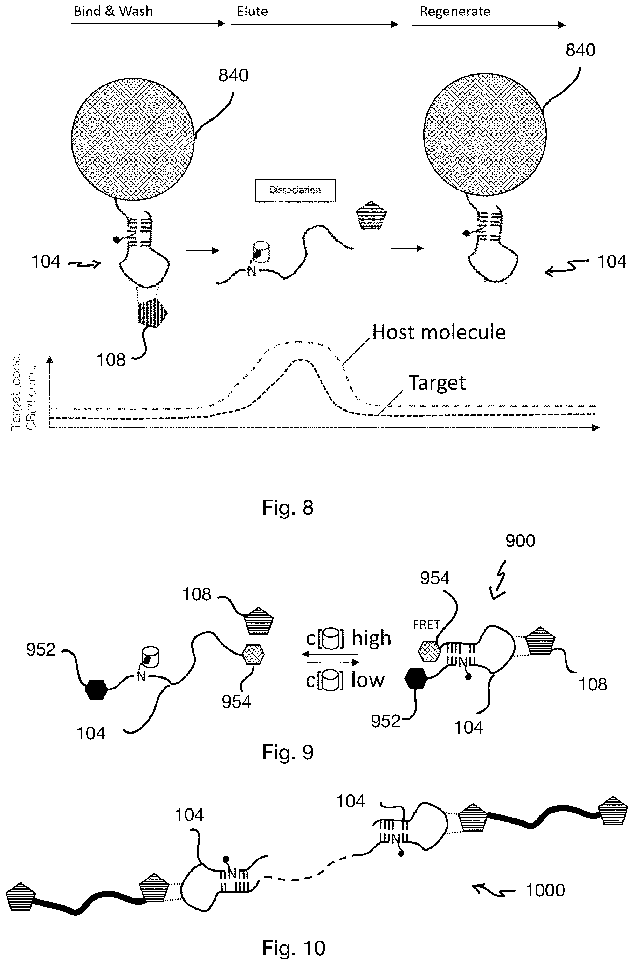

Figure 8 illustrates an example in which the affinity reagent 104 including the guest-modified aptamer 104 is used for affinity purification. In this example, it may be assumed that the guest-modified aptamer 106 serves to bind the molecular target 108 to an affinity matrix 840 which may be a material used for chromatography columns, e.g. in FPLC or HPLC systems. The matrix 840 may also be used for benchtop applications such as spinning down beads or spin-through columns which allow protein purification on a smaller scale using benchtop centrifuges.

The characteristic in the lower part of Figure 8 indicates that the host molecule concentration c is increased at a time when the molecular target 108 is to be removed from the matrix 840. The characteristic further indicates that a concentration of the molecular target 108 increases at the same time.

The example shown in Figure 8 allows for gentle elution of the bound molecular target 108 and gentle regeneration of the column or matrix as opposed to melting aptamers at a high temperature, which usually damages the sample and/or the column matrix. Similarly, the affinity reagent 104 may be introduced into magnetic beads which are used to purify molecular targets or even cell.

Figure 9 illustrates an example in which the affinity reagent 104 is used in a FRET sensor 900 which includes two sensing dyes 952, 954. In case that the molecular target 108 is bound to the affinity reagent 104 of the FRET sensor 900, the sensing dyes 952, 954 are brought close to each other, allowing an energy transfer according to the FRET principle. Based on the energy transfer, the presence of the molecular target 108 can be detected.

Figure 10 illustrates an example in which multiple affinity reagent 104 are used to build a responsive biomaterial 1000. In this example, the guest-modified aptamers of affinity reagents 104 are multimerized on nucleic acid or protein backbones. The affinity reagents 104 serve to bind and unbind other components of the biomaterial 1000. For example, a hybrid-polymer may be generated in this way, which can be liquified and solidified depending on the presence of the host molecule.

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### List of Reference Signs

- 100: host molecule
- 102: guest molecule
- 104: affinity reagent
- 106: aptamer
- 108: molecular target
- 110: transient zone
- 840: affinity matrix
- 900: FRET sensor
- 952, 954: sensing dyes
- 1000: responsive biomaterial

## Claims

1. An affinity reagent (104) for reversibly binding to a molecular target (108), comprising a guest-modified aptamer (106) which includes at least one guest molecule (102) adapted for complex formation with at least one host molecule (100), the guest-modified aptamer (106) being structurally alterable by said complex formation to either associate with or dissociate from the molecular target (108).

2. The affinity reagent (104) according to claim 1, wherein the guest-modified aptamer (106) includes natural nucleic acids or synthetic nucleic acids.

3. The affinity reagent (104) according to one of the preceding claims, wherein the guest-modified aptamer (106) has a single-stranded structure which is alterable by said complex formation between a folded state and an unfolded state.

4. The affinity reagent (104) according to one of the preceding claims, wherein the guest-modified aptamer (106) is configured to associate with the molecular target (108) in the folded state and to dissociate from the molecular target (108) in the unfolded state.

5. The affinity reagent (104) according to one of the preceding claims, wherein the guest molecule (106) of the guest-modified aptamer (104) is adapted for complex formation with a cucurbituril.

6. The affinity reagent (104) according to one of the preceding claims, further comprising at least one detectable label (412, 414, 612, 614).

7. A structure comprising a plurality of affinity reagents (104) according to one of the preceding claims, wherein the structure includes at least one of a DNA nanostructure, a gel, a polymer, a chromatography resin, and a magnetic particle.

8. A device comprising a plurality of affinity reagents (104) according to one of the preceding claims, wherein the device comprises a FRET sensor (900).

9. A reagent kit comprising at least one host molecule (100) and at least one affinity reagent (104) for reversibly binding to a molecular target (108), the at least one affinity reagent (104) comprising a guest-modified aptamer (106) which includes at least one guest molecule (102) adapted for complex formation with the at least one host molecule (100), the guest-modified aptamer (106) being structurally alterable by said complex formation to either associate with or dissociate from the molecular target (108).

10. The reagent kit according to claim 9, further comprising a solid support or a flow cell.

11. A method for reversibly binding affinity reagents (104) to molecular targets (108), each affinity reagent (104) comprising a guest-modified aptamer (106) which includes at least one guest molecule (102) adapted for complex formation with at least one host molecule (100), the guest-modified aptamer (106) being structurally alterable by said complex formation to either associate with or dissociate from the respective molecular target (108),
wherein the method comprises the following steps:
associating the affinity reagents (104) with the molecular targets (108) at a first concentration of the host molecules (100), and
dissociating the affinity reagents (104) from the molecular targets (108) at a second concentration of the host molecules (100), the second concentration being different from the first concentration.

12. The method according to claim 11, further comprising a step of detecting at least one label (412, 414, 612, 614) included in each affinity reagent (104) after the affinity reagent (104) is associated with the molecular target (108) and before the affinity reagent (104) is dissociated from the molecular target (108).

13. The method according to claim 12, further comprising a step of removing the affinity reagents (104) from a sample containing the molecular targets (108) after the affinity reagents (104) are dissociated from the molecular targets (108).

14. The method according to claims 11 to 13, wherein a sequence of the steps of associating, detecting, dissociating, and removing is cyclically repeated.
